Europäisches Patentamt

European Patent Office

Office européen des brevets

(19)

(11) Veröffentlichungsnummer : **0 119 156**
**B1**

(12) EUROPÄISCHE PATENTSCHRIFT

(45) Veröffentlichungstag der Patentschrift :
11.11.87

(51) Int. Cl.⁴ : **A 61 K 47/00**, A 61 K 31/557

(21) Anmeldenummer : 84730011.8

(22) Anmeldetag : 10.02.84

(54) Prostaglandin-haltiges pharmazeutisches Präparat und seine Herstellung.

(30) Priorität : 02.03.83 DE 3307816

(43) Veröffentlichungstag der Anmeldung :
19.09.84 Patentblatt 84/38

(45) Bekanntmachung des Hinweises auf die Patenterteilung : 11.11.87 Patentblatt 87/46

(84) Benannte Vertragsstaaten :
DE FR GB IT

(56) Entgegenhaltungen :
DE-A- 711 440
DE-A- 3 001 454
FR-A- 2 311 530
CHEMICAL ABSTRACTS, Band 86, Nr. 22, 30. Mai
1977, Seite 376, Nr. 161302r, Columbus, Ohio, US;
CHEMICAL ABSTRACTS, Band 95, Nr. 4, 27. Juli 1981,
Seite 341, Nr. 30415t, Columbus, Ohio, US;

(73) Patentinhaber : SCHERING AKTIENGESELLSCHAFT
Berlin und Bergkamen
Müllerstrasse 170/178 Postfach 65 03 11
D-1000 Berlin 65 (DE)

(72) Erfinder : Raptis, Georg, Dr.
Miquelstrasse 77
D-1000 Berlin 33 (DE)
Erfinder : Gädke, Horst-Werner
Genter Strasse 2
D-1000 Berlin 65 (DE)

## Beschreibung

Die Erfindung betrifft Lyophilisate von Alkalipolyacrylaten, die mit wäßrigen Lösungen von Prostaglandinen aufgequollen werden, und ihre Verwendung.

Natürliche Prostaglandine und ihre synthetischen Analoga sind wirksame Mittel für die Cervixerweiterung.

Aus der DE-OS 3 001 454 sind Prostaglandinhaltige pharmazeutische Formulierungen bekannt. Diese Formulierungen stellen Lyophilisate von Hydroxylgruppen-haltigen Polymeren mit Prostaglandinen dar, die vor der Applikation in gebrauchsfertige viskose Lösungen umgewandelt werden. Häufig bilden sich dabei Klümpchen, die die Freisetzung des Wirkstoffes erschweren oder sogar verhindern.

Die vorliegende Erfindung betrifft Lyophilisate von Alkalipolyacrylaten, die mit wäßrigen Lösungen von Prostaglandinen versetzt werden.

Die in Wasser suspendierte Polyacrylsäure wird mit einer 0,1-1 n, vorzugsweise 0,5 n Alkalihydroxidlösung auf einen pH-Wert von 5-7, vorzugsweise 6-6,5 eingestellt, 20-60 Minuten bei 120-130 °C vorzugsweise 20-30 Minuten bei 120-125 °C im Autoklaven (1-1,5 Bar Überdruck) sterilisiert und aseptisch gefriergetrocknet. Dieses Lyophilisat ist verschlossen unbegrenzt haltbar. Für die galenische Formulierung eines Prostaglandins für die Cervixerweiterung wird dieses Lyophilisat mit einer wäßrigen Lösung des Prostaglandins (50-200 µg Prostaglandin in 5-20 ml destilliertem Wasser) versetzt und durch kurzes Schütteln mit der Hand in eine gebrauchsfertige viskose Lösung überführt.

Als Alkalihydroxid kommen sowohl Natriumals auch Kaliumhydroxid in Frage.

Als Prostaglandine kommen alle für die Cervixerweiterung geeigneten Prostaglandine in Betracht, vorzugsweise Prostaglandine aus der E-Reihe.

Die neue Formulierung enthält weniger fremde Bestandteile als die bekannten Formulierungen. Außerdem ist sie einfacher herzustellen. Während die bekannten Zubereitungen infolge von Klümpchenbildung zu wenig befriedigenden Cervixerweiterung führen, wird der Wirkstoff aus der vorliegenden Formulierung schnell und vollständig freigesetzt, so daß eine einwandfreie Medikation gewährleistet ist.

Die Erfindung betrifft somit ein Prostaglandinhaltiges Arzneimittel, das vor jeder intravaginalen und/oder intrazervikalen Anwendung aus einem Lyophilisat eines Alkalipolyacrylats und einer wäßrigen Lösung eines Prostaglandins zubereitet wird.

In den nachfolgenden Beispielen wird die Erfindung weiter erläutert, ohne sie zu beschränken.

Beispiel 1

2,5 g Carbopol® 934 werden in 497,5 g destilliertem Wasser suspendiert und mit 0,5 n Natriumhydroxid-Lösung auf einem pH-Wert 6 eingestellt. Die viskose Lösung wird in 20 ml-Flaschen abgefüllt und bei 120 °C 20 Minuten im Autoklaven sterilisiert. Die sterilen Lösungen werden aseptisch gefriergetrocknet, verschlossen und gelagert. Zur Herstellung der gebrauchsfertigen Formulierung wird der Inhalt einer Ampulle mit 100 µg des Wirkstoffes 16-Phenoxy-prostaglandin-$E_2$-methansulfonamid in 10 ml destilliertem Wasser aufgenommen und in die Flasche mit dem Trockengel gegeben. Nach kurzem Schütteln ist die Mischung gebrauchsfertig.

Beispiel 2

0,25 g Indigo Carmin (Blauer Farbstoff) und 2,5 g Carbopol® 934 werden in 497,25 g destilliertes Wasser gegeben. Unter Zugabe von 10 ml 0,5 Natriumhydroxidlösung stellt sich ein pH-Wert von 6 ein das suspendierte Carbopol geht in Lösung.

Die entstandene viskose Lösung wird in 20 ml-Flaschen abgefüllt und bei 120 °C 20 Minuten im Autoklaven sterilisiert. Die sterilen Lösungen werden aseptisch gefriergetrocknet, verschlossen und gelagert. Zur Herstellung der gebrauchsfertigen Formulierung wird der Inhalt einer Ampulle mit 100 µg des Wirkstoffes 16-Phenoxy-prostaglandin-$E_2$-methansulfonamid in 10 ml destilliertem Wasser aufgenommen und in die Flasche mit dem Trockengel gegeben. Nach kurzem Schütteln ist die Mischung gebrauchsfertig.

## Patentansprüche

1. Prostaglandin-haltiges Arzneimittel für intravaginale und/oder intrazervikale Anwendung enthaltend Alkalipolyacrylat-Lyophilisate, die mit wäßrigen Lösungen vom Prostaglandinen versetzt werden.

2. Arzneimittel gemäß Anspruch 1, dadurch gekennzeichnet, daß das verwendete Alkalipolyacrylat ein Natriumpolyacrylat ist.

3. Arzneimittel gemäß Anspruch 1 bis 2 dadurch gekennzeichnet, daß das Prostaglandin ein Prostaglandin-$E_2$-derivat ist.

4. Arzneimittel gemäß Anspruch 2 bis 3 dadurch gekennzeichnet, daß das Prostaglandin 16-Phenoxy-prostaglandin-$E_2$-methansulnamid.

5. Verfahren zur Herstellung des Prostaglandin-haltigen Arzneimittels gemäß Anspruch 1, dadurch gekennzeichnet, daß man ein Lyophilisat aus Alkalipolyacrylat mit einer wäßrigen Prostaglandinlösung versetzt.

## Claims

1. Prostaglandin-containing pharmaceutical composition for intravaginal and/or intracervical use containing alkali-metal polyacrylate lyophili- sates which are treated with aqueous solutions of the prostaglandin.

2. Pharmaceutical composition according to claim 1, characterised in that the alkali-metal polyacrylate used is a sodium polyacrylate.

3. Pharmaceutical composition according to claim 1 or 2, characterised in that the prostaglandin is a prostaglandin-$E_2$ derivative.

4. Pharmaceutical composition according to claim 2 or 3, characterised in that the prostaglandin is 16-phenoxyprostaglandin-$E_2$ methanesulphonamide.

5. Method for the preparation of the prostaglandin-containing pharmaceutical composition according to claim 1, characterised in that a lyophilisate of an alkali-metal polyacrylate is treated with an aqueous prostaglandin solution.

**Revendications**

1. Médicament contenant une prostaglandine et destiné à être appliqué par la voie intravaginale et/ou intra-cervicale, médicament qui contient un lyophilisat de poly-(acrylate de métal alcalin) auquel on ajoute une solution aqueuse d'une prostaglandine.

2. Médicament selon la revendication 1 caractérisé en ce que le poly-(acrylate de métal alcalin) utilisé est un poly-(acrylate de sodium).

3. Médicament selon l'une des revendications 1 et 2, caractérisé en ce que la prostaglandine est un dérivé de la prostaglandine-E2.

4. Médicament selon l'une des revendications 2 et 3, caractérisé en ce que la prostaglandine est le phénoxy-16 prostaglandine-E2-méthane-sulfonamide.

5. Procédé de préparation d'un médicament contenant une prostaglandine, selon la revendication 1, procédé caractérisé en ce qu'on ajoute une solution aqueuse de prostaglandine à un lyophilisat d'un poly-(acrylate de métal alcalin).